# EUROPEAN PATENT APPLICATION

(11) **EP 4 530 300 A1**
(43) Date of publication of application: **02.04.2025**
(21) Application number: 22943010.3
(22) Date of filing: 23.05.2022
(51) Int. Cl.: C07K 19/00, C12N 15/62, A61P 31/20, A61P 31/14, A61P 31/16, A61K 38/17, A61K 45/06, A61K 38/21

(54) **TREFOIL FACTOR 2/INTERFERON ALPHA2 FUSION PROTEIN AND APPLICATION THEREOF IN PREVENTION AND TREATMENT OF VIRAL INFECTIOUS DISEASES**

(71) Applicant: Fudan University, Shanghai 200433 (CN)
(72) Inventor: XU, Jianqing, Shanghai 200433 (CN); ZHANG, Xiaoyan, Shanghai 200433 (CN); ZHAI, Guanxing, Shanghai 200433 (CN)
(74) Representative: V.O.
(86) International application number: PCT/CN2022/094509
(87) International publication number: WO 2023/225802

(57) **Abstract**

The present disclosure provides a trefoil factor 2 (TFF2)/interferon α2 (IFNα2) fusion protein and an application thereof in prevention and treatment of viral infectious diseases. Specifically, the present disclosure provides a fusion protein, comprising: a TFF2 element containing a TFF2 peptide, and an IFNα2 element fused with the TFF2 element and containing an IFNα2 peptide. The present disclosure also provides an application of the fusion protein in prevention and/or treatment of viral infectious diseases (e.g., acute respiratory/enterovirus infections).

## Description

### TECHNICAL FIELD

. The present application belongs to the field of biomedical technology and specifically relates to a fusion protein of trefoil factor 2 (TFF2) and interferon α2 (IFNα2), and preparation and use thereof in the treatment and prevention of viral infection diseases.

### BACKGROUND

. Viral infections cause great harm to the life and health of humans and mammals, among which respiratory viral infections and enteroviral infections are the most common acute viral infections.

. Acute respiratory viral infections are prone to cause epidemics, which seriously threaten human life and health, and have also had an adverse impact on people's lives and socio-economic development; and therefore, there is an urgent need to develop prevention and control measures for similar epidemics. Enteroviruses are a class of sense single-stranded RNA viruses associated with human and mammalian diseases that are transmitted through the intestines. Enteroviruses affect millions of people worldwide each year and are usually present in the respiratory secretions (such as saliva, sputum, or nasal mucus) and feces of infected people. Infection can cause a variety of symptoms, including mild respiratory illness (common cold), hand, foot and mouth disease, acute hemorrhagic conjunctivitis, aseptic meningitis, myocarditis, severe neonatal sepsis-like disease, acute flaccid paralysis, and related acute flaccid myelitis.

. Both respiratory viruses and enteroviruses are mucosal infecting viruses. Although the characteristics and infection ways of different viruses are not completely consistent, their common pathogenic mechanisms can still be traced. On the one hand, the direct viral infection causes apoptosis or necrosis of target cells and damages the structure and function of normal mucosa; on the other hand, viruses inhibit the production of type I interferon and its signaling pathway by regulating the host immune response, which induces a large amount of inflammatory cytokines and chemokine secretions, produces an inflammatory storm, recruits a large number of immune cells to infiltrate the mucosal tissue. These severely damage the mucosal structure, cause mucosal damage, inflammatory exudation, *etc.,* and ultimately cause diseases or death in a host. Therefore, referring to the common pathogenic mechanisms of respiratory and enteric viral infections, it is necessary to suppress the inflammatory storm and promote the repair of mucosal damage while limiting viral replication in order to achieve the purpose of improving patient prognosis.

. Currently, the commonly used anti-inflammatory drug for the inflammatory response induced by viral infection is glucocorticoid, which is a class of steroid hormones. Glucocorticoids are part of the feedback mechanism of the immune system and can reduce certain aspects of immune function, thereby effectively suppressing inflammation. However, its side effects are also obvious. The currently used glucocorticoid drugs have non-selective effects and can damage many healthy anabolic processes. The side effects of long-term use of this drug include iatrogenic hyperadrenocortical syndrome, inducing or aggravating infection or causing potential metastasis of infection lesions *in vivo,* causing peptic ulcers, inducing pancreatitis and fatty liver, iatrogenic adrenocortical insufficiency, inducing schizophrenia and epilepsy, femoral head necrosis, *etc.*

. As an antiviral drug, interferon has been widely used in various viral infections when used alone or in combination with other therapies. And it has inhibitory effects on many currently known viruses. As a classic antiviral drug, IFNα2 has been widely used in clinical practice, wherein the α-2b spray thereof has significant efficacy in the treatment of acute upper respiratory tract infections in children and can effectively improve the clinical symptoms and signs of children *(*Chen Qing et al., Analysis of the efficacy of recombinant human interferon α-2b spray in the treatment of acute upper respiratory tract infection in children, Biomedical Engineering and Clinical. 2019, 23(04*));* the α-2b spray at a low dose of 20 µg in combination with Oseltamivir for the treatment of Influenza A can downregulate the levels of cellular inflammatory cytokines, increase the virus clearance rate, promote symptom improvement, and is of high safety *(*Xu Guangfeng , Evaluation of the efficacy of recombinant human interferon α-2b spray combined with oseltamivir in the treatment of influenza A, Shanxi Health Journal of the College of Health Professions. 2020,30(04*)).* Both animal experiments and clinical studies have shown that the combination of IFNα2 and Ribavirin can effectively inhibit the replication of MERS-CoV and improve MERS symptoms and clinical outcomes. In the 2003 SARS treatment plan and the latest version of the "Novel Coronavirus Pneumonia Diagnosis and Treatment Plan (Trial Eighth Edition)", it is recommended to use IFNα2 treatment (5,000,000 U or equivalent dose per administration for adults, adding 2 mL of sterile water for injection, twice daily via aerosol inhalation, and the treatment course not exceeding 10 days). Anuja Pandit conducted a phase II study of pegylated interferon alpha-2b (PEG IFN-α2b) in moderate COVID-19 in 2020 to evaluate its efficacy and safety, wherein a single subcutaneous dose of 1 µg/kg PEG IFN-α2b + standard treatment can reduce the duration of viral disappearance and significantly improve clinical outcomes compared with standard treatment *(*Anuja Pandit, Efficacy and safety of pegylated interferon alfa-2b in moderate COVID -19: A phase II, randomized, controlled, open-label study, Int J Infect Dis. 202104;105:516-521*).*

. As a small molecule peptide secreted by the gastrointestinal tract, TFF2 can participate in mucosal repair. Although it is over-expressed during inflammation, adding TFF2 will have an anti-inflammatory effect, help create a microenvironment required for tissue repair, and promote tissue repair *(*Abdelaziz Ghanemi et al., Trefoil factor family member 2 (TFF2) as an inflammatory-induced and anti-inflammatory tissue repair factor, Animals (Basel). 2020 Sep 14;10(9):1646*).* In previous studies, we found that TFF2, a host-secreted peptide, can reduce pathological damage, promote lung tissue damage repair, exert a protective effect, and improve the prognosis of influenza virus infection by inhibiting inflammatory responses (CN105582526B). In the COVID-19 clinical study launched urgently in 2019, we combined TFF2 with type I interferon kappa (IFN-κ) for treating patients with moderate COVID-19 pneumonia via aerosol inhalation. The results showed that this combination treatment could significantly shorten the time for COVID-19 patients to become negative for nucleic acid tests, increase the proportion of patients who become negative for nucleic acid tests, speed up the improvement of patients' CT scans, and reduce the length of hospitalization of patients. Meantime, the levels of inflammatory cytokines in the plasma of treated patients decreased rapidly (EClinicalMedicine 2020: 100478/100547; Chinese Patent Application No. 202010239633.3).

. In the long-term struggle between humans and viral infections, there is still an urgent need to develop safer and more effective drugs and methods to prevent and treat viral infections.

### SUMMARY

. The present disclosure provides an effective active substance that can be used more safely and effectively for the prevention and/or treatment of viral infections, its use in the manufacture of a medicament, and methods for preventing and treating diseases.

. In one aspect of the present disclosure, a fusion protein is provided, which comprises one or more fusion units, each comprising:
(a) a trefoil factor 2 (TFF2) element, wherein the TFF2 element comprises a TFF2 peptide or an active fragment thereof;
(b) an interferon α2 (IFNα2) element, wherein the IFNα2 element comprises an IFNα2 peptide or an active fragment thereof,
wherein the TFF2 element and the IFNα2 element are fused at a molecular ratio of 1:1, and in each fusion unit the TFF2 element is located at the N-terminus of the IFNα2 element.

. In some embodiments, the TFF2 peptide or an active fragment thereof is derived from humans, primates, rodents (e.g., mice, rats, guinea pigs, hamsters), dogs, or cats.

. In some embodiments, the TFF2 peptide or an active fragment thereof comprises an amino acid sequence selected from the group consisting of SEQ ID NO: 8 and SEQ ID NO: 12, or an active fragment thereof (e.g., an amino acid sequence that has at least 80% sequence identity with SEQ ID NO: 8 and SEQ ID NO: 12 and has TFF2 activity).

. In some embodiments, the TFF2 peptide or an active fragment thereof is encoded by a nucleic acid molecule comprising a nucleotide sequence selected from the group consisting of SEQ ID NO: 7 and SEQ ID NO: 11, or an active fragment thereof (e.g., a nucleic acid molecule having at least 80% sequence identity with SEQ ID NO: 7 or SEQ ID NO: 11 and capable of encoding an active TFF2 peptide).

. In some embodiments, the IFNα2 peptide or an active fragment thereof is derived from humans, primates, rodents (e.g., mice, rats, guinea pigs, hamsters), dogs, or cats.

. In some embodiments, the IFNα2 peptide or an active fragment thereof comprises an amino acid sequence selected from the group consisting of SEQ ID NO: 10 and SEQ ID NO: 14, or an active fragment thereof (e.g., an amino acid sequence having at least 80% sequence identity with SEQ ID NO: 10 and SEQ ID NO: 14 and having IFNα2 activity).

. In some embodiments, the IFNα2 peptide or an active fragment thereof is encoded by a nucleic acid molecule comprising a nucleotide sequence selected from the group consisting of SEQ ID NO: 9 and SEQ ID NO: 13, or an active fragment thereof (e.g., a nucleic acid molecule having at least 80% sequence identity with SEQ ID NO: 9 or SEQ ID NO: 13 and capable of encoding an active IFNα2 peptide).

. In some embodiments, the fusion protein further comprises a linker that connects the TFF2 element and the IFNα2 element and/or the constituent peptide segments in the element.

. In some embodiments, the linker is a flexible linker comprising n amino acid residues, where n is an integer of 2-300.

. In some embodiments, the linker is a glycine or glycine/serine linker. In some embodiments, the linker is selected from the amino acid sequence of Gn, (GS)n, (GGS)n, (GGGS)n, (GGGGS)n, or (GGGGGS)n, wherein n is an integer of 1, 2, 3, 4, 5, 6, 7, 8, 9, or 10.

. In some embodiments, the fusion protein comprises one or more TFF2 peptides arranged continuously or intermittently and/or one or more IFNα2 peptides arranged continuously or intermittently.

. In some embodiments, the fusion protein further comprises an Fc region, wherein the Fc region does not contain mutations. In some embodiments, the fusion protein comprises one or more mutations that reduce antibody-mediated ADCC and CDC activity. In some embodiments, the fusion protein comprises amino acid mutations D265A and N297G according to EU numbering.

. In some embodiments, the fusion protein further comprises a signal peptide. In some embodiments, the signal peptide in the fusion protein is selected from the tPA2 signal peptide, TFF2 signal peptide, IL-2 signal peptide, bPRL signal peptide, CD33 signal peptide.

. In some embodiments, the fusion protein further comprises a label, for example, a label for purification, detection, or localization, such as a label selected from fluorescent labels, non-radioactive nuclide labels, biotin labels, phosphorylation modification labels, and peptide tags.

. In some embodiments, the fusion protein has an amino acid sequence of SEQ ID NO: 2 or has at least 80% sequence identity thereto. In some embodiments, the fusion protein is encoded by a nucleic acid molecule having a nucleotide sequence of SEQ ID NO: 1 or a nucleic acid molecule having at least 80% sequence identity thereto.

. In some aspects of the present disclosure, an isolated nucleic acid molecule or a construct or vector comprising the nucleic acid molecule is provided, wherein the nucleic acid molecule encodes a fusion protein of the present disclosure. In some embodiments, the nucleic acid molecule has a nucleotide sequence of SEQ ID NO: 1 or has at least 80% sequence identity thereto. In some embodiments, the nucleic acid molecule encodes a polypeptide having an amino acid sequence of SEQ ID NO: 2 or having at least 80% sequence identity thereto.

. In some embodiments, the vector is selected from a viral vector, an mRNA vector, and a DNA vector.

. In some aspects of the present disclosure, a cell is provided, which comprises the fusion protein of any one of claims 1 to 7, or the nucleic acid molecule, construct, or vector of claims 8 or 9.

. In some aspects of the present disclosure, a composition is provided, which comprises the fusion protein, nucleic acid molecule, construct, vector, and/or cell of the present disclosure; and a carrier.

. In some aspects of the present disclosure, provided is the use of the fusion protein, nucleic acid molecule, construct, vector, cell, and/or composition of the present disclosure in the preparation of a medicament for preventing and/or treating viral infectious disease.

. In some aspects of the present disclosure, a method for preventing and/or treating a viral infectious disease is provided, the method comprises administering to a subject in need thereof an effective amount of the fusion protein, nucleic acid molecule, construct, vector, cell, composition or a medicament comprising the foregoing substances of the present disclosure.

**.** In some aspects of the present disclosure, provided are the fusion proteins, nucleic acid molecules, constructs, vectors, cells, compositions, and/or drugs of the present disclosure for use in preventing and/or treating viral infectious diseases.

. In some embodiments, the viral infectious disease is selected from acute viral infection, such as respiratory viral infection and enteroviral infection.

. In some embodiments, the viral infectious disease is caused by one or more viruses selected from the group consisting of coronavirus, influenza virus, rhinovirus, adenovirus, parainfluenza virus, respiratory syncytial virus, coxsackievirus, echovirus, and novel enterovirus.

. In some embodiments, the fusion proteins, nucleic acid molecules, constructs, vectors, cells, compositions, and/or medicaments of the present disclosure are administered as prophylactic drugs before a viral infection occurs to prevent the occurrence of a viral infection or to reduce the severity of a subsequent viral infection.

. In some embodiments, the fusion proteins, nucleic acid molecules, constructs, vectors, cells, compositions, and/or medicaments of the present disclosure are administered as therapeutic drugs after viral infection occurs to reduce the severity of viral infection and disease.

. In some embodiments, the fusion proteins, nucleic acid molecules, constructs, vectors, cells, compositions, and/or medicaments of the present disclosure are used as both preventive and therapeutic drugs and are administered continuously or intermittently before and after viral infection occurs.

. In some embodiments, the fusion protein, nucleic acid molecule, construct, vector, cell, composition, and/or medicaments of the present disclosure is in a form suitable for administration selected from the group consisting of respiratory tract atomization inhalation, nasal drops, spray, oral administration, intramuscular injection and/or intravenous administration.

. In some embodiments, the fusion proteins, nucleic acid molecules, constructs, vectors, cells, compositions, and/or medicaments of the present disclosure are suitable for use alone or in combination with other antiviral drugs, immune drugs, or viral therapies.

. Those skilled in the art may arbitrarily combine the above technical solutions and technical features without departing from the inventive concept and protection scope of the present invention. Other aspects of the present invention are obvious to those skilled in the art based on the disclosure of the present application.

### DESCRIPTION OF DRAWINGS

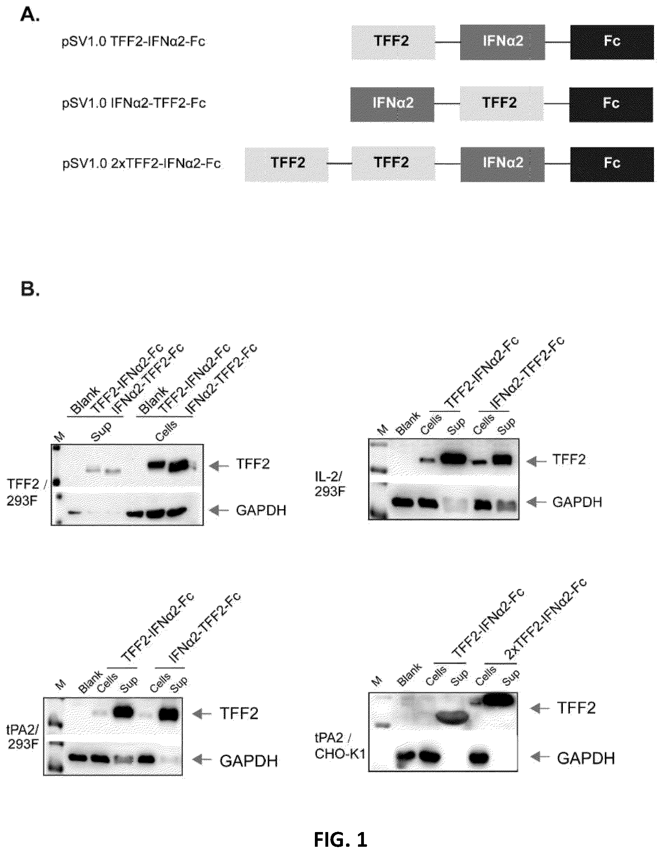

**.** The present invention will be further illustrated below in conjunction with the accompanying drawings, and these drawings are only for illustrating the embodiments of the present invention rather than limiting the scope of the present invention.
. **FIG. 1****:** Construction and *in vitro* expression of fusion protein expression vector:
   **FIG. 1A****:** Construction map of the eukaryotic expression vectors pSV1.0 IFNα2-TFF2-Fc, pSV1.0 TFF2-IFNα2-Fc and pSV1.0 2xTFF2-IFNα2-Fc of TFF2 and IFNα2 fusion proteins;
   **FIG. 1B****:** Expression of TFF2 and IFNα2 fusion proteins with different signal peptides in 293F suspension cell lines and CHO-K1 cells, wherein "Blank" represents the control cell, "Cell" represents the cell, "Sup" represents the supernatant, and the signal peptides are TFF2, IL-2 signal peptide, and tPA2 signal peptide, respectively.
. **FIG. 2****:** Validation of the fusion protein of TFF2 and IFNα2 and its effects on viral protein expression and replication.
   **FIG. 2A****:** Verification of the fusion protein of TFF2 and IFNα2 on polyacrylamide gel electrophoresis (PAGE) after purification;
   **FIGS. 2B & 2C****:** Effects of TFF2 and IFNα2 fusion protein on the expression of interferon-induced transmembrane protein 3 (IFITM3) protein in lung epithelial cell line A549;
   **FIGS. 2D & 2E****:** Effect of TFF2 and IFNα2 fusion protein on the replication of influenza virus PR8 in the lung epithelial cell line A549 *in vitro.*
. **FIG. 3****:** Effects of TFF2 and IFNα2 fusion protein on the expression of virus-induced inflammatory-related factors.
   **FIG. 3A****:** Effect of TFF2 and IFNα2 fusion protein on PR8-induced COX-2 expression;
   **FIG. 3B****:** Effect of TFF2 and IFNα2 fusion protein on the expression of IL-6 induced by PR8;
   **FIG. 3C****:** Effect of TFF2 and IFNα2 fusion protein on LPS-induced iNOS expression in the lung epithelial cell line A549 *in vitro.*
   *** denotes p < 0.001.
. **FIG. 4****:** Protective effects of TFF2-IFNα2-Fc and IFNα2-TFF2-Fc on animals challenged with influenza virus.
**FIG. 4A****:** Viral challenge and administration model for TFF2-IFNα2-Fc, IFNα2-TFF2-Fc influenza virus ;
**FIG. 4B****:** Survival rate curve and body weight change curve of mice after PR8 infection (TFF2-IFNα2-Fc);
**FIG. 4C****:** Survival rate curve and body weight change curve of mice after PR8 infection (IFNα2-TFF2-Fc);
**FIG. 4D****:** Viral challenge and administration model for TFF2-IFNα2-Fc, IFNα2-TFF2-Fc influenza virus ;
**FIG. 4E****:** Survival curve of mice after PR8 infection (TFF2-IFNα2-Fc, 2xTFF2-IFNα2-Fc);
**FIG. 4F****:** Body weight change curve of mice after PR8 infection (TFF2-IFNα2-Fc, 2xTFF2-IFNα2-Fc).

### DETAILED DESCRIPTION

. Through long-term and in-depth research, the inventors have constructed various forms of TFF2 and IFNα2 fusion proteins. After testing, comparison, and screening of the antiviral, inflammation-reducing, animal protection effects and other functions of individual TFF2 or IFNα2 polypeptides and various fusion proteins, as well as overcoming various technical difficulties, the inventors obtained specific fusion protein forms with correct structure and excellent effects. Therefore, the present disclosure provides a TFF2 and IFNα2 fusion protein with a specific structure and ratio; and further verifies its excellent preventive and therapeutic effects on viral infectious diseases. The fusion protein of the present application has both an excellent antiviral effect and the effect of inhibiting excessive inflammation, and its effect is significantly better than that of TFF2 or IFNα2 alone, which provides a synergistic effect and is also significantly better than that of other TFF2 and IFNα2 fusion proteins with other structures and ratios.

. Specifically, in the present disclosure, TFF2 polypeptide and IFNα2 polypeptide were fused and expressed in various forms, the successful expression of the fusion protein was verified through *in vitro* experiments, and the inhibitory effect of TFF2 and IFNα2 fusion protein on influenza virus replication and its effect on decreasing the secretion of inflammatory factors were tested. Further studies in the mouse influenza infection model found that aerosol inhalation of TFF2 and IFNα2 fusion protein could improve the survival rate of influenza-infected mice and reduce the weight loss of mice. Among the various fusion proteins constructed, the fusion protein containing TFF2 polypeptide and IFNα2 polypeptide at a molecular ratio of 1:1 and with the TFF2 polypeptide located at the N-terminus of IFNα2 has the most excellent effect. The test results show that the anti-viral and excessive inflammation-inhibiting effects of such a specific type of fusion protein *in vivo* and *in vitro* are significantly better than those of TFF2 or IFNα2 alone, which provides a synergistic effect, and are also significantly better than that of other TFF2 and IFNα2 fusion proteins with other structures and ratios.

**.** More specifically, in order to develop new antiviral drugs and verify their effects in the prevention and treatment of antiviral infections, the inventors constructed eukaryotic expression vectors pSV1.0 IFNα2-TFF2-Fc, pSV1.0 TFF2-IFNα2-Fc, and pSV1.0 2xTFF2-IFNα2-Fc. These vectors were expressed in 293T and the expression of fusion proteins was verified by Western Blot; also, they were expressed in 293F cells, and the supernatant was collected and then purified by ÄKTA pure, and further, the purity of the purified protein was identified by Coomassie Brilliant Blue, and the protein concentration was determined by BCA. In the *in vitro* cell experiment, Western blot was used to detect the effect of TFF2 and IFNα2 fusion protein on the replication of influenza virus PR8 in the lung epithelial cell line A549 *in vitro,* proving that the fusion protein could reduce the replication of the virus.

. For mice infected with influenza virus HIN1 strain PR8 strain, the weight changes and survival rate of PR8 infected mice were observed. It was found that the TFF2 and IFNα2 fusion protein treatment group could significantly improve the survival rate of mice, reduce weight loss, and improve the symptoms of highly pathogenic influenza infection. On this basis, we conducted a preventive experiment with TFF2 and IFNα2 fusion protein to further verify its preventive and protective effects against influenza virus H1N1 strain PR8 strain challenge. These results fully prove that TFF2 and IFNα2 fusion protein plays an important protective role in acute viral infection injury models induced by respiratory viruses or other factors.

. Since the mechanism of tissue damage caused by other acute viral infections is similar to that of influenza virus infection, the protective effect of TFF2 and IFNα2 fusion protein is not limited to respiratory tissue damage caused by the influenza virus, but also includes respiratory damage caused by other acute viral infections, as well as intestinal diseases such as enterovirus-induced acute symptoms. In addition, preventive administration to disease control personnel and high-risk groups involved in the management of acute viral infection epidemics can effectively reduce the risks and damages suffered by the above personnel.

. In summary, this application is further improved and optimized based on previous research. By fusing TFF2 and IFNα2 in a specific form, it can exert multi-molecule and multifunctional effects at the same time, and the effect is significantly better than giving a single drug separately. The formation of a single molecule through fusion can not only play a multifunctional role, but also reduce the complexity of multi-protein combinations, improve efficacy, and play a dual role of antiviral and anti-inflammatory effects. Through the fusion protein of TFF2 and IFNα2 in a single molecule, the antiviral, anti-inflammatory, and protective functions of the fusion protein in viral infectious diseases, especially acute viral infectious diseases, were analyzed. The specific fusion protein of the present disclosure targets the common pathogenic mechanism of acute viral infection. On the one hand, it can exert the broad-spectrum inhibition function of IFNα2 on viral replication; on the other hand, it can inhibit inflammatory cytokine storm through TFF2, which promotes mucosal damage repair, improves prognosis, and exerts antiviral and repair-promoting effects. Meantime, the two fusion components complement each other and unexpectedly exert an effect that is significantly better than what can be obtained by administrating the two components alone. Adjusting the expression ratio of TFF2 and IFNα2 in the fusion protein helps to eliminate the side effects of IFNα2-induced inflammatory cytokine storm and achieve safe and effective intervention.

. In addition, TFF2 is a small molecule host polypeptide that is highly conserved in different species. For example, the mature human TFF2 molecule consists of 106 amino acids, with a molecular weight of about 12kD, wherein it comprises two symmetrical special conserved sequences consisting of about 40 amino acid residues, and three intrachain disulfide bonds formed by 6 cysteine residues (cys1-cys5, cys2-cys4 and cys3-cys6), thereby producing a specific and stable clover-shaped structure, which is acid-resistant, heat-resistant and resistant to protease hydrolysis, and has great advantages in transportation. IFNα2 is also a polypeptide secreted by the host and has been widely used in clinical practice. Therefore, the fusion expression of TFF2 and IFNα2 not only has clear activity and good safety, but also has a high drugability rate, and has a high application potential in the prevention and treatment of acute respiratory virus infections and enterovirus infections.

. The intervention strategy of TFF2 and IFNα2 fusion protein is proposed for the first time in this disclosure, which adopts the three-in-one strategy of antiviral + anti-inflammatory + pro-repair effects, and can be used for broad-spectrum prevention and treatment of acute infectious diseases caused by viral infection. The fusion protein can not only be administered systemically, but also can be administered by local atomization inhalation, which is highly targeted, effective, and has few side effects, and can exert better effects. In addition, the product disclosed in this disclosure has low cost, is easy for popularization and application in economically weak countries or regions, and can also be used as a technical reserve for the national prevention and control of infectious diseases caused by new emerging viruses. Therefore, it has high economic value, social value, and political significance.

. The present disclosure provides a fusion protein of trefoil factor 2 and interferon α2. Such fusion protein has the advantages of inhibiting viral replication, reducing tissue inflammation and tissue damage, and promoting lung tissue function repair. It can be used to prepare medicament for treating and/or preventing acute viral infection diseases. The medicament also has a significant improvement effect on the prognosis caused by acute viral infection.

. Compared with the prior arts, the present disclosure provides a fusion protein based on TFF2 and IFNα2. The inventors have proved through experiments that the TFF2 and IFNα2 fusion protein molecules can play a protective role in influenza virus PR8 infection, reduce morbidity and mortality, and alleviate respiratory tract infection inflammation symptoms, thereby playing an important role in responding to the epidemic of new respiratory tract viral infections, especially for the prevention and treatment of viral infections and severe infections for which there are no effective therapeutic drugs. TFF2 and IFNα2 fusion proteins target the common pathogenic mechanisms of acute viral infections and play an antiviral role by inhibiting inflammatory response, promoting mucosal tissue repair, reducing tissue damage, and inhibiting the replication function of the virus. Therefore, the protective effect of TFF2 and IFNα2 fusion proteins is not limited to respiratory tissue damage caused by influenza viruses, including damage caused by other viral infections. Preventive administration to disease control personnel and high-risk groups involved in the treatment of acute viral infection epidemics can effectively reduce the risks and damages suffered by the above-mentioned personnel.

. All numerical ranges provided herein are intended to clearly include all values falling between the range endpoints and the numerical ranges therebetween. Features mentioned in the present application or the embodiments may be combined. All features disclosed in the present application may be used in combination with any composition form, and each feature disclosed in the present application may be replaced by any alternative feature that can provide the same, equal, or similar purpose. Unless otherwise specified, the disclosed features are only general examples of equal or similar features.

. As used herein, "comprising", "having", or "including" encompasses "consisting of", "consisting essentially of", and "comprised of"; "consisting essentially of" "substantially consisting of" and "consisted of" are sub-concepts of "comprising", "having", or "including".

. As used herein, "mammal(s)" may include humans, primates, rodents (e.g., rats, mice, guinea pigs, hamsters), domestic animals, or farm mammals.

### TFF2 element and IFNα2 element

. As used herein, the term "element" refers to an amino acid sequence that constitutes a part of a fusion protein. The term "unit" refers to a basic fragment of an element that constitutes a function. For example, the TFF2 element of a fusion protein may comprise one or more continuous or intermittent TFF2 units, each of which can produce the desired TFF2-related functional activity.

. As used herein, the terms "TFF2 element" and "TFF2 protein (polypeptide)" are used interchangeably and refer to a natural (e.g., mammalian origin), recombinant, or synthetic TFF2 polypeptide sequence that constitutes a part of a fusion protein. The natural TFF2 is highly conserved in mammals, has a specific and stable clover-shaped structure, and has certain mucosal repair and inflammation inhibition effects. TFF2 polypeptides also comprise natural variants and fragments of TFF2 (e.g., splice variants or allelic variants), as well as non-naturally occurring variants having natural TFF2 activity.

. The nucleotide and amino acid sequences of native TFF2 polypeptides are known in the art. See, for example, GenBank accession Gene ID: 7032 (human) and 21785 (murine).

. The TFF2 element disclosed herein may comprise the amino acid sequence of SEQ ID NO: 8 (human TFF2) or be encoded by a nucleic acid molecule comprising SEQ ID NO: 7, comprise the amino acid sequence of SEQ ID NO: 12 (mouse TFF2) or be encoded by a nucleic acid molecule comprising SEQ ID NO: 11, or may be a homologous sequence (for example, a homologous sequence can be obtained through a database or alignment software known in the art), a variant or a modified form having the same or similar activity as these proteins. For example, the TFF2 polypeptide may be selected from (a) a polypeptide comprising an amino acid sequence set forth in SEQ ID NO: 8 or 12 (for example, a polypeptide having a sequence as shown in SEQ ID NO: 8 or 12); or (b) a protein or polypeptide derived from (a) that has one or more amino acids substituted, deleted or added in the amino acid sequence defined in (a) and has the activity of mucosal repair and inflammation inhibition.

. As used herein, the terms "IFNα2 (polypeptide)" and "IFNα2 protein (polypeptide)" are used interchangeably and refer to natural (e.g., mammalian origin), recombinant or synthetic IFNα2 polypeptides. As described in the background section, the structure and function of IFNα2 have been studied and understood to a certain extent in the art. In the present application, IFNα2 polypeptides known in the art may be used, as well as natural variants and fragments thereof (e.g., splice variants or allelic variants), and non-natural variants having IFNα2 activity.

. The nucleotide and amino acid sequences of the native IFNα2 polypeptide are known in the art. *See, for example,* GenBank accession Gene ID: 3440 (human) and 15965 (murine).

. The IFNα2 protein used herein may comprise the amino acid sequence of SEQ ID NO: 10 (human IFNα2) or be encoded by a nucleic acid molecule comprising SEQ ID NO: 9, may comprise the amino acid sequence of SEQ ID NO: 14 (mouse IFNα2) or be encoded by a nucleic acid molecule comprising SEQ ID NO: 13, or may be a homologous sequence (e.g., a homologous sequence can be obtained through a database or alignment software known in the art), a variant or a modified form having the same or similar activity as these proteins. For example, the IFNα2 polypeptide may be selected from (a) a polypeptide comprising the amino acid sequence of SEQ ID NO: 10 or 14 (e.g., a polypeptide having a sequence as shown in SEQ ID NO: 10 or 14); or (b) a protein or polypeptide derived from (a) that has one or more amino acids substituted, deleted or added in the amino acid sequence defined in (a) and has the activity of mucosal repair and inflammation inhibition.

. The fusion protein of the TFF2 polypeptide element and the IFNα2 polypeptide element of the present disclosure are preferably encoded by human genes or homologous genes or family genes thereof. The variant forms of proteins or polypeptides disclosed in the present disclosure include (but are not limited to) one or more (usually 1 to 50, preferably 1 to 30, more preferably 1 to 20, and most preferably 1 to 10, such as 1, 2, 3, 4, 5, 6, 7, 8, 9 or 10) amino acid deletions, insertions and/or substitutions, and the addition of one or more (usually within 20, preferably within 10, and more preferably within 5) amino acids at the C-terminus and/or N-terminus. For example, in the art, when amino acids with similar or similar properties are substituted, the function of the protein or polypeptide is usually not changed. For another example, the addition of one or more amino acids at the C-terminus and/or N-terminus usually does not change the function of the protein or polypeptide, for example, the fusion protein may or may not include the initial methionine residue but still have its desired viral infection prevention and treatment activity.

. Variant forms of polypeptides include homologous sequences, conservative variants, allelic variants, natural mutants, induced mutants, and proteins encoded by sequences that can hybridize with their protein-coding sequences under high or low stringency conditions. Depending on the host used in the recombinant production scheme, the protein or polypeptide of the present invention can be glycosylated or non-glycosylated.

### Fusion Protein

. As used herein, the term "fusion protein" refers to an amino acid molecule comprising at least one TFF2 polypeptide and at least one IFNα2 polypeptide fused together. The disclosed fusion protein can be produced from a prokaryotic or eukaryotic host (e.g., bacteria, yeast, higher animals, insects, and mammalian cells; preferably a eukaryotic host) using recombinant technology, or can be produced by artificial synthesis, such as by full sequence synthesis or fragment synthesis followed by splicing.

**.** The TFF2 peptide in the fusion protein herein can be linked to the N-terminus or C-terminus of the IFNα2 peptide, preferably the TFF2 peptide can be linked to the N-terminus (i.e., upstream) of the IFNα2 peptide. The fusion protein herein can comprise one or more TFF2 peptides and/or IFNα2 peptides, for example, the TFF2 peptide and the IFNα2 peptide are fused at a molecular ratio of 5:1 to 1:5, such as a molecular ratio of 1:1 or 2:1, preferably a molecular ratio of 1:1.

. In a preferred embodiment of the present application, the TFF2 peptide in the fusion protein herein is linked to the N-terminus of the IFNα2 peptide, and the TFF2 peptide and the IFNα2 peptide are fused at a molecular ratio of 1:1.

. The fusion protein of the present application may also comprise an Fc region. As used herein, the term "Fc region" or "Fc fragment" refers to the immunoglobulin Fc segment used in the fusion protein. In some embodiments, the Fc region has an amino acid sequence substantially identical to a natural or variant immunoglobulin Fc fragment, and has substantially the same biological activity as a natural Fc fragment. In addition to the CH2 and CH3 regions of an immunoglobulin, the Fc region may also include a hinge region. The Fc region may be derived from, for example, IgG or IgA.

. Similar to the function of the Fc segment in monoclonal antibodies, the Fc segment of the fusion protein can extend the half-life of the functional protein in plasma, improve the stability of the molecule, specifically bind to the Fc receptor *in vivo,* and exert corresponding biological functions. In addition, the Fc segment can specifically bind to protein A, thereby simplifying the purification step of the Fc fusion protein, which is of great significance in the research and development (R&D) and preparation of related biological products. The Fc region used in the fusion protein herein may not comprise mutations, or may comprise one or more mutations, such as mutations that reduce antibody- mediated ADCC and CDC activity, such as amino acid mutations D265A and N297G/N297Q according to the EU numbering.

. The fusion protein herein may also comprise a signal peptide, such as an amino acid sequence having the function of guiding the secretion, localization, and/or delivery of the fusion protein, which is generally 5-50 amino acids in length. In some embodiments, the signal peptide may be selected from, for example, tPA2 signal peptide, TFF2 signal peptide, IL-2 signal peptide, bPRL signal peptide, CD33 protein signal peptide, etc.

. The fusion protein herein may further comprise a label, for example, a label for purification, detection, or localization, such as a fluorescent label, a non-radioactive nuclide label, a biotin label, a phosphorylation modification label, or a peptide tag.

. Each polypeptide element in the fusion protein herein or peptide unit in the element can be connected by a linker. In the present application, a flexible linker is preferably used so that there can be interaction between the polypeptide elements or peptide units. The linker that can be used in the fusion protein of the present application can contain 2 to 300 amino acid residues, such as 5 to 100, 10 to 50, and 15 to 3 amino acid residues. Exemplary linkers can be glycine linkers, such as (G)n, or glycine/serine linkers, such as amino acid sequences of (GS)n, (GGS)n, (GGGS)n, (GGGGS)n or (GGGGGS)n, wherein n is an integer of 1, 2, 3, 4, 5, 6, 7, 8, 9 or 10.

. Based on the sequences provided in the present application and the techniques in the art, the fusion protein of the present invention can be easily prepared by various known methods by those skilled in the art. These methods include but are not limited to, recombinant DNA methods, artificial synthesis, etc. (See Murray KM, Dahl SL Ann; Pharmacother 1997 Nov; 31(11): 1335-8). For example, the fusion protein herein can be produced by direct peptide synthesis using solid-phase technology, or each fragment of the fusion protein can be chemically synthesized separately and then chemically linked to produce a full-length molecule.

### Vector and host

. The present disclosure also relates to vectors for producing fusion proteins, and host cells produced by genetic engineering using the vectors.

. The coding sequence of the present invention can be used to express or produce a recombinant fusion protein by conventional recombinant DNA technology (Science, 1984; 224: 1431). Generally speaking, the following steps are involved:
(1) transforming or transducing a suitable host cell with a polynucleotide (or variant) encoding a fusion protein as described herein, or with a recombinant expression vector containing the polynucleotide;
(2) culturing host cells in a suitable culture medium;
(3) isolating and purifying the target fusion protein from the culture medium or cells.

. In the present invention, the terms "vector" and "recombinant expression vector" are used interchangeably and refer to bacterial plasmids, bacteriophages, yeast plasmids, animal cell viruses, mammalian cell viruses, or other vectors well-known in the art, which can replicate in host cells and express target proteins.

. Methods well known to those skilled in the art can be used to construct expression vectors containing fusion protein coding sequences and suitable transcription/translation control signals. These methods include *in vitro* recombinant DNA technology, DNA synthesis technology, *in vivo* recombination technology, etc. The DNA sequence can be effectively linked to an appropriate promoter in the expression vector to guide mRNA synthesis. The expression vector also comprises a transcription terminator and a ribosome binding site for translation initiation. pSV1.0 vectors, pcDNA3.1 vectors, pIRES2-EGFP vectors, and AdMaxTM expression systems can be used herein.

. In addition, the expression vector preferably comprises one or more selectable marker genes to provide a phenotypic trait for the selection of transformed host cells, such as dihydrofolate reductase, neomycin resistance, and green fluorescent protein (GFP) for eukaryotic cell culture, or tetracycline or ampicillin resistance for *Escherichia coli.*

. The vector containing the above-mentioned appropriate DNA sequence and appropriate promoter or control sequence can be used to transform appropriate host cells to enable them to express proteins or polypeptides. The host cell can be a prokaryotic cell, such as a bacterial cell; or a lower eukaryotic cell, such as a yeast cell; or a higher eukaryotic cell, such as an animal cell. Representative examples include animal cells, such as 293F cells, CHO cells, etc.; *Escherichia coli,* Streptomyces genus, Agrobacterium genus; fungal cells, such as yeast.

. When the polynucleotide of the present invention is expressed in higher eukaryotic cells, transcription will be enhanced if an enhancer is inserted into the vector. The enhancer is a cis-acting factor of DNA, usually about 10 to 300 base pairs, which act on promoters to enhance gene transcription. Those skilled in the art are aware of how to select appropriate vectors, promoters, enhancers, and host cells.

. By the above methods, the fusion protein can be expressed in the cell or on the cell membrane or secreted outside the cell. If necessary, the recombinant protein can be separated and purified by various separation methods based on its physical, chemical, and other properties. These methods are well known to those skilled in the art. Examples of these methods include but are not limited to conventional renaturation treatment, treatment with a protein precipitant (salting out method), centrifugation, ÄKTA pure method, ultra treatment, ultracentrifugation, molecular sieve chromatography (gel filtration), adsorption chromatography, ion exchange chromatography, high-performance liquid chromatography (HPLC) and various other liquid chromatography techniques and combinations thereof.

### Medicament/drug or kit

. Also provided herein is a product comprising an effective amount of the fusion protein, a vector comprising the encoding molecule for the fusion protein, host cells described herein, as well as a pharmaceutically or physiologically acceptable carrier. As used herein, the term "active ingredient" refers to the fusion protein described herein, its encoding nucleic acid molecule, the construct or vector comprising the nucleic acid molecule, host cells, or an aforementioned composition.

. In a preferred embodiment, the products herein can be used to prevent or treat diseases and/or symptoms associated with viral infections. As used herein, the terms "containing" or "comprising" encompass "comprising", "consisting essentially of", and "consisting of". As used herein, the term "pharmaceutically acceptable" ingredient(s) refers to substances that are suitable for use in humans and/or animals without causing excessive adverse side effects (such as toxicity, irritation, and allergic reactions), that is, they have a reasonable benefit/risk ratio. As used herein, the term "effective amount" refers to an amount that can produce a function or activity in humans and/or animals and can be accepted by humans and/or animals.

. As used herein, the term "pharmaceutically acceptable carrier" refers to a carrier for the administration of a therapeutic agent, including various excipients and diluents. The term refers to pharmaceutical carriers that are not essential active ingredients themselves and are not excessively toxic after administration. Suitable carriers are well known to those skilled in the art. A full discussion of pharmaceutically acceptable excipients can be found in "Remington's Pharmaceutical Sciences" (Mack Pub. Co., N.J., 1991).

. The pharmaceutically acceptable carrier in the composition may comprise a liquid such as water, saline, glycerol, and/or ethanol. In addition, auxiliary substances may also be present in these carriers, such as fillers, disintegrants, lubricants, glidants, effervescent agents, wetting agents or emulsifiers, flavoring agents, pH buffer substances, etc. Generally, these substances can be formulated in a non-toxic, inert, and pharmaceutically acceptable aqueous medium, wherein the pH is generally about 5 to 8, preferably, the pH is about 6 to 8.

. As used herein, the term "unit dosage form" refers to a dosage form prepared for easy administration in a single dose required for each application, including but not limited to various solid forms (such as tablets, freeze-dried powders), liquid forms (such as solutions), capsule forms, and controlled-release forms.

. In another preferred embodiment of the invention, the composition is in unit dosage form or a multi-dose form, and the content of the active ingredient therein ranges from 0.01 to 2000 mg per dose, preferably 0.1 to 1500 mg per dose, more preferably 1 to 1000 mg per dose. In another preferred example of the invention, 1 to 6 doses of the composition of the invention are administered per day, preferably 1 to 3 doses; most preferably, the daily dosage is 1 dose.

. The pharmaceutical composition of the invention can be formulated into various dosage forms as needed and the beneficial dosage for patients can be determined by physicians based on factors such as the type of patient, age, weight, general health condition, mode of administration, etc. And the pharmaceutical composition of the invention can be administered by respiratory tract atomization inhalation, nasal drops, spray, oral administration, intramuscular injection, and/or intravenous administration.

. In order to improve the therapeutic effects, the active substances or products of the present invention can be used in combination with each other, and can also be combined with other medicaments/drugs and treatment methods, for the prevention and treatment of infectious diseases (and especially acute viral infections). For example, if the fusion protein of the present invention is used to prevent and/or treat acute viral infections, other medicaments/drugs or methods clinically used for the treatment of acute viral infections can be used simultaneously or sequentially, and the other drugs or methods include but are not limited to preventing further damage, regulating local regional functions, anti-inflammation, administering glucocorticoids, non-steroidal anti- inflammatory drugs NSAIDs, etc.

### EXAMPLES

. In conjunction with specific examples, this application is further elucidated below. It should be understood that these examples are provided for illustration only and are not intended to limit the scope of this application. Those skilled in the art may make appropriate modifications and changes to the present invention, and these modifications and changes are all within the scope of the present invention.

. Experimental methods in the following examples, wherein specific conditions are not mentioned, can adopt conventional methods in the field, such as those referenced in "Molecular Cloning: A Laboratory Manual" (3rd edition, Cold Spring Harbor Laboratory Press, New York, 1989) or according to conditions recommended by suppliers. DNA sequencing methods are conventional in the field and can also be provided by commercial companies.

. Unless otherwise stated, percentages and parts are calculated by weight. Unless otherwise defined, all technical and scientific terms used herein have the same meanings as those familiar to those skilled in the art. In addition, any methods and materials similar or equivalent to those described herein can be applied to the methods of the present invention. The preferred implementation methods and materials described herein are for demonstration purposes only.

### Example 1. Construction and design of fusion protein plasmid and eukaryotic expression

**.** Within the present example, gene sequences were first cloned according to the amino acid sequence of human TFF2 (e.g., SEQ ID NO: 8) and the amino acid sequence of human IFNα2 protein (e.g., SEQ ID NO: 10), and the recombinant plasmids of different forms were transfected into 293T cells. The eukaryotic expression of TFF2 and IFNα2 fusion protein was then detected by Western Blot (WB), and then a large amount of expression was performed in 293F cells. The expression supernatant was harvested and purified by the HiTrap MabSelect SuRe column. After the target protein was collected, the purity was identified, and ultrafiltration was replaced with PBS to obtain a high-purity TFF2 and IFNα2 fusion protein. The specific steps are as follows:

. The human TFF2 sequence was linked to interferon α 2 through three G4S, and TFF2 had different repeat clones and a human Fc fragment was added at the end (FIG. 1A). The correctly sequenced recombinant plasmid was transfected into 293T cells, wherein the transfection reagent was TurboFect, and the culture medium was DMEM complete culture medium (10% FBS and 1% P/S). The cells were taken out after 24 h of incubation at 37°C, followed by collecting the cells and adding an SDS loading buffer. After transferring the cells to EP tubes and washing the cells with PBS buffer, the loading buffer was added and heated in a boiling water bath for 10 minutes to denature the proteins. After instant centrifugation, the proteins were separated by SDS-polyacrylamide gel electrophoresis (SDS-PAGE), and the separation gel concentration was 10%. The electrophoresis voltage was 70 V, and the time was 30-40 minutes (marked by the start of separation of the marker). After bromophenol blue migrated to the separation gel position, the voltage was adjusted to 110 V until bromophenol blue migrated to the bottom of the gel, and then the membrane was quickly transferred at a constant current of 400 mA for 50 minutes. After the transfer, the PVDF front membrane (the surface in contact with the gel) was marked and blocked in 5% skim milk powder at room temperature for 2 hours. Then, the primary antibody (TFF2, Proteintech: 13681-1-AP, 1:1000; IFNα2, SantaCruz, sc-73305, 1:1000; β-actin, ABclonal, AC028, 1:5000) at an appropriate dilution ratio was added, diluted with 5% skim milk powder, and incubated overnight at 4°C on a shaker. After washing the membrane with 0.05% PBST, the secondary antibody (goat anti-rabbit (1:5000); goat anti-mouse (1:5000)) was added and diluted with 5% skim milk powder in PBST. After incubation on a shaker at room temperature for 1 hour, the membrane was washed and developed by ECL. The PVDF membrane was exposed to a quantitative analyzer for 2 minutes, and the color development results were recorded and analyzed.

. The results showed **(****FIG. 1B****,** upper left) that the cells expressed a large amount of TFF2 and IFN α2 fusion protein. When the TFF2 signal peptide was used, the fusion protein was mainly expressed in the cells, and the other part was secreted into the supernatant.

. In order to obtain a large amount of secreted fusion protein, we replaced the signal peptides for the TFF2 and IFN α 2 recombinant plasmids, wherein the TFF2 signal peptide itself was replaced with the IL-2 signal peptide (FIG. 1B, upper right) and tPA2 signal peptide (**FIG**. 1B lower left and lower right). By comparing the supernatant secretions of different signal peptides, we found that the tPA2 signal peptide can more effectively promote the secretion of the fusion protein. Therefore, the tPA2 signal peptide was used in the subsequent fusion protein expression.

### Example 2: Purification of fusion protein and antiviral and anti-inflammatory functions in vitro

. To obtain a large amount of recombinant protein, the correctly sequenced TFF2 and IFNα2 recombinant plasmids were transfected into 293F suspension cells. Specifically, 293F cells were suspended and cultured in SMM 293-TI serum-free medium, supplemented with 1% penicillin/streptomycin antibiotics. 5x10 ⁵ cells/mL were inoculated into fresh medium each time, and experiments or passages were performed when the density reached 3x10 ⁶ cells/mL or above. The cell shaker was aerated with 5% carbon dioxide and rotated at a speed of 125 rpm/min. The cell density was 1x10 ⁶ cells/mL when the fusion protein plasmid was transiently transfected. PEI was used for transfection, and the ratio of DNA: PEI during transfection was 1:3.5. An appropriate amount of plasmid was placed in a 1.5 mL EP tube and diluted to 40 ng/µL with 150 mM sodium chloride. After mixing, PEI was added. After vortexing and mixing thoroughly, the mixture was incubated at room temperature for 15-30 min. The DNA/PEI mix was added to the cells. After 24 hours, an anti-cell clumping agent (Anti-Clumping Agent, Thermo Fisher, 0010057AE) was added at a ratio of 1: 1000. The cells were cultured on a cell shaker for 5-7 days and the supernatant and cells were collected. The WB detection showed that the fusion protein was mainly secreted in the supernatant, and therefore the supernatant was directly used for purification.

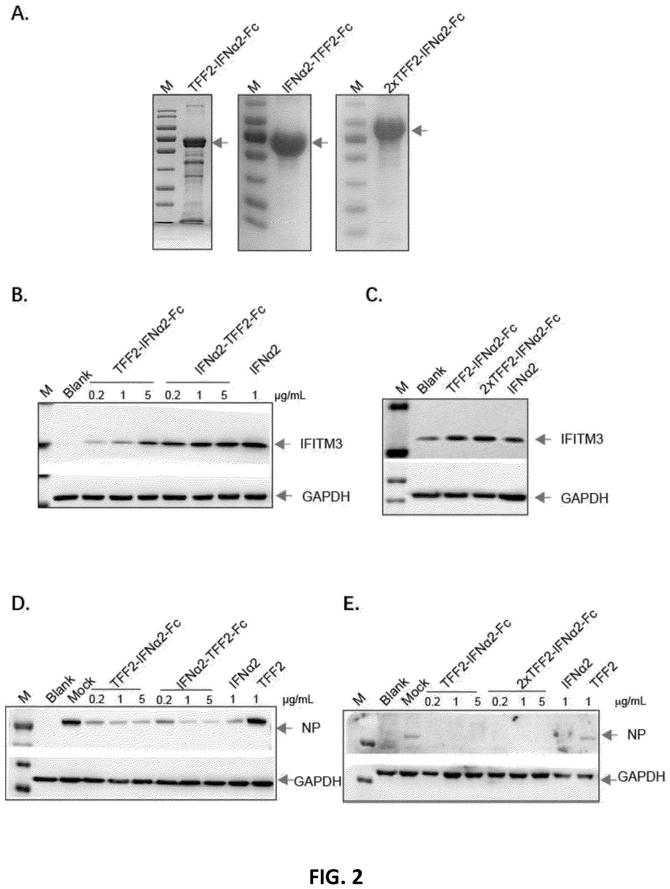

. The fusion protein was purified using the protein purifier ÄKTA Pure, and the column was HiTrap MabSelect SuRe 1 mL column. The operation was performed by running UNICORN software. All buffers were filtered through a 0.22 µm filter membrane. First, the pump and the pipeline were cleaned with ultrapure water, then the column was connected and cleaned, and the ethanol was rinsed with at least 5 volumes of distilled water. The column was equilibrated through the A1 pipeline with binding buffer (0.02 M sodium phosphate, 0.15 M NaCl, pH 7.2), and the B1 pump was filled with elution buffer (0.1 M sodium citrate, pH 3.0). After equilibration, the sample was loaded through the pipeline A1. And the sample was also centrifuged at 12000xg and filtered through a 0.22 µm filter membrane before loading. The sample was loaded at a rate of 0.5 mL/min, and the flow-through was collected at the same time. After loading, a binding buffer was used to wash until the baseline was flat, and then switched to B1 to elute with 100% elution. The eluate was collected until the baseline was basically flat, and 100-200 µL of neutralizing solution (Tris-HCl, pH 9.0) was added to each 1.5 mL EP tube. Then, the column was eluted and regenerated with 5 column volumes (CV) of elution buffer, followed by 3 CV of binding buffer, 5 CV of 0.1 to 0.5 M NaOH to wash the column, and then 5 to 10 CV of binding buffer was used for re-equilibration. The column was rinsed with 20% ethanol and stored at 4°C. For the purified fusion protein, it is replaced to PBS buffer through a 10 kDa ultrafiltration centrifuge tube. In order to quantify the purity of TFF2 and IFNα2 fusion protein, SDS-PAGE was performed according to the above method, and then stained with Quick Blue fast staining solution for 30-60 min and washed with ddH₂O overnight (FIG. 2A). In order to quantify the concentration of TFF2 and IFNα2 fusion protein, a BSA standard curve was made using the BCA quantitative kit. The concentration of TFF2 and IFNα2 fusion protein was quantified based on the optical density (OD) value of the standard, and the protein was aliquoted and frozen at -80 degrees.

. The interferon activity experiment of the fusion protein was conducted in A549 cells. The cells were plated the day before, with 1x10⁵ cells per well in a 24-well plate. The fusion protein was added, and after 24 hours, the cells were washed once with PBS. Trypsin digestion was performed for 2 to 3 minutes, and the culture medium was terminated. The cells were then detached by gentle pipetting, washed again with PBS, and 100 µL of 1x loading buffer was added. The samples were subjected to a boiling bath for 10 minutes, and the expression of interferon-induced transmembrane protein 3 (IFITM3) was detected by WB analysis **(****FIG. 2B****).** IFITM3 belongs to the interferon-induced gene ISG, which can be induced by interferon and virus. The fusion protein can stimulate the expression of IFITM3, indicating that the fusion protein has the function of inducing interferon activity. Different orders of TFF2 and IFNα2 fusion have different *in vitro* activities. IFNα2-TFF2-Fc can induce strong expression of IFITM3, while TFF2-IFNα2-Fc and 2xTFF2-IFNα2-Fc have slightly weaker IFITM3 expression levels. Subsequent *in vitro* and *in vivo* experiments were further combined to determine whether the interferon-inducing activity of the fusion protein is moderate.

. The antiviral experiment of the fusion protein was conducted in A549 cells. The cells were plated the day before, with 1x10⁵ cells per well in a 24-well plate. The fusion protein was added 4-6 hours in advance. On the following day, the cells were infected with a multiplicity of infection (MOI) of 5. After a 2-hour infection period, the cells were washed once with PBS and the medium was replaced with DMEM containing 10% FBS. After 24 hours, the cells were washed once with PBS, trypsin digestion was performed for 2 to 3 minutes, and the culture medium was terminated. The cells were then detached by gentle pipetting, washed again with PBS, and 100 µL of 1x loading buffer was added. The samples were subjected to a boiling bath for 10 minutes, and the expression of nucleoprotein (NP) was detected by WB analysis **(****FIG. 2C****).** The results showed that fusion protein can reduce the replication of the virus, and the antiviral effect gradually increased with the increase of fusion protein concentration, while TFF2 alone could not affect the replication of the virus.

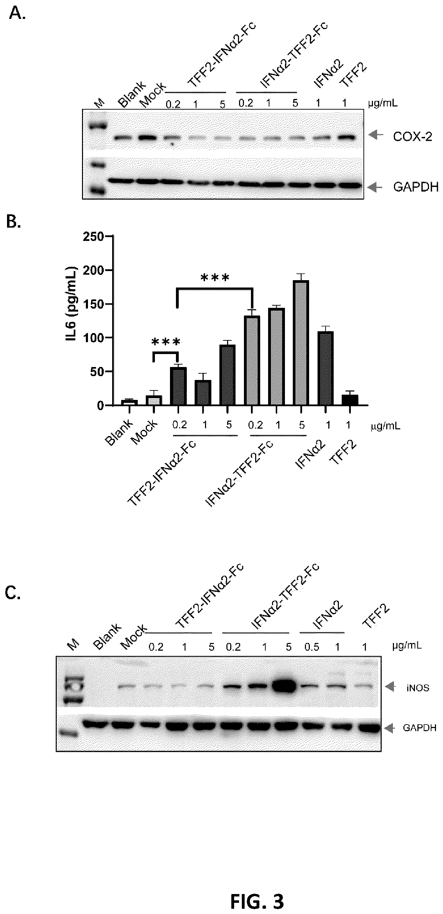

. The expression of COX-2 protein was detected by WB **(****FIG. 3A****).** The activity of COX-2 in normal tissue cells is extremely low. When cells are stimulated by inflammation, their expression level in inflammatory cells can increase to 10 to 80 times the normal level. In the experiment, the expression level of COX-2 in A549 cells was significantly increased under the influence of PR8 virus, proving that TFF2-IFNα2-Fc and IFNα2-TFF2-Fc fusion proteins can inhibit the replication of PR8 while reducing the expression of cyclooxygenase COX-2. IFNα2 alone can also decrease the levels of cyclooxygenase COX-2, but TFF2 alone is not capable of inhibiting viral replication and therefore cannot reduce the levels of COX-2 induced by the virus.

. The expression of inflammatory factor IL-6 in the supernatant after PR8 infection was detected by ELISA in A549 cells (FIG. 3B). The cells were plated, with 1x10⁵ cells per well in a 24-well plate, 12 wells, and supernatants were collected after 24 h. Before the test, the plate was coated with ELISA coating solution precooled at 4°C (ELISA coating solution: 10 mM sodium carbonate (Na₂CO₃), 30 mM sodium bicarbonate (NaHCO₃), pH = 9.6, sterilized by filtration with a 0.2 µm filter membrane, and stored at 4°C). 100 µL of capture antibody solution diluted 1:250 was added to each well of the ELISA plate and stored at 4°C overnight. The inflammatory factor standard protein was lyophilized powder. First, the lyophilized powder was dissolved with sterile distilled water according to the label instructions, and a serial dilution was performed with the standard diluent. The stock solution concentration after complete dissolution was 1000 pg/mL. The standard was gently shaken for 5 min before dilution. On the second day, the plate was washed three times with 300 µL/well wash buffer (PBS with 0.05% Tween-20) and blocked with ELISA blocking buffer (PBS with 10% FBS) for 1 h. Sample loading: 100 µL/ well of diluted cytokine standards were added, with a concentration gradient of standards as follows: 500 pg/mL, 250 pg/mL, 125 pg/mL, 62.5 pg/mL, 31.25 pg/mL, 15.6 pg/mL, 7.8 pg/mL; and serial dilution of the standards was performed in imported EP tubes. Then, 100 µL/well of sample was added to the wells. After incubation of samples and standards for 2h, they were washed 5 times with wash buffer. Adding detection antibody: A diluted 100 µL detection antibody + SAv-HRP reagent was added to each well at 100 µL/well. Incubation was performed at room temperature for 1 hour. The liquid in the wells was washed off, and the wash buffer stayed for 1 minute before being discarded from the wells, this process was repeated 7 times, and finally, they were inverted and dried on a piece of filter paper. Development: 100 µL/well of TMB was added and incubated at room temperature in the dark for 30 min. Terminating the reaction: 100 µL/well of stop solution was quickly added to terminate the reaction. Plate reading: the absorbance at 450 nm was measured within 10 minutes after the addition of the stop solution. The corresponding concentration on the coordinate was determined based on the absorbance values of the samples.

. These results showed that compared with the IFNα2 µg/mL treatment group, the TFF2-IFNα2-Fc fusion protein at doses of 0.2 and 1 µg/mL can significantly reduce the PR8-induced IL-6 inflammatory levels, and at a dose of 5 µg/mL, it was comparable to the secreted IL-6 inflammatory levels in IFNα2 treatment. Compared with IFNα2, IFNα2-TFF2-Fc can induce higher IL-6 inflammation levels in a dose-dependent manner. Although IFNα2 itself has antiviral effects, the level of IL-6 inflammation also appears under its action, indicating that different orders of TFF2 and IFNα2 can affect the IL-6 inflammation level induced by IFNα2. In RAW264.7 cells, the cells were plated the day before, at 2.5x10 ⁵ cells/well in a 12-well plate, and the fusion protein was added 4 to 6 hours in advance. After incubating with the fusion protein at concentrations of 0.2, 1, and 5 µg/mL for 4 hours, 1 ng/mL of LPS was added. After 24 hours, the cells were washed once with PBS. Trypsin digestion was performed for 2 to 3 minutes, and the culture medium was terminated. The cells were then detached by gentle pipetting, washed again with PBS, and 100 µL 1x loading buffer was added. The samples were subjected to a boiling bath for 10 minutes, and the expression of iNOS was detected by WB. Under LPS stimulation of RAW264.7 cells, TFF2-IFNα2-Fc produces lower iNOS than IFNα2 (FIG. **3C****).**

. The level of iNOS may be an indicator of the degree of inflammation in the body. The results show that compared with IFNα2, TFF2-IFNα2-Fc can significantly reduce the expression of iNOS. IFNα2-TFF2-Fc can promote the expression level of inflammation, and as the dose increases, it gradually induces an increase in the expression level of iNOS. The structure of IFNα2 in the center within TFF2-IFNα2-Fc can affect the function of IFNα2, and IFNα2 can induce higher levels of iNOS. The structure of IFNα2 in the center can reduce the level of its induced iNOS, while the structure of IFNα2 in front within IFNα2-TFF2-Fc does not affect the function of IFNα2. Therefore, as the dose increases, the level of induced iNOS also increases. This result shows that TFF2-IFNα2-Fc fusion protein can induce appropriate iNOS levels and reduce the risk of excessive inflammatory response.

### Example 3: Animal challenge protection and prevention experiments of fusion proteins

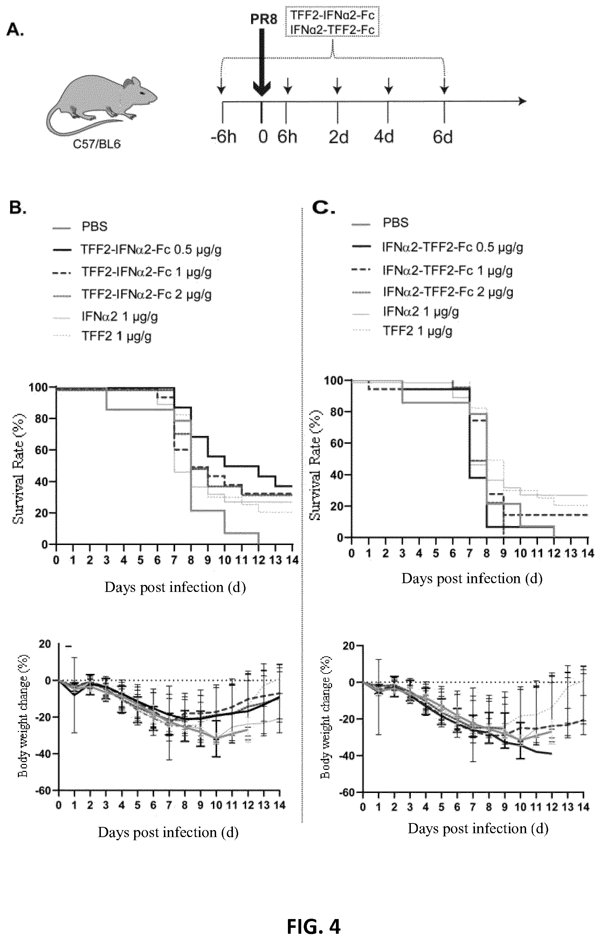

. In this example, influenza virus H1N1 strain PR8 (in a P2 laboratory) was used to infect C57 mice via intranasal administration. The fusion protein was administered 6 h prior to infection **(****FIG. 4A****).** Before infection, 0.5 g of Tribromoethanol + 1 mL of 2-methyl-2-butanol + 39 mL of water was used to prepare a 40 mL solution to anesthetize mice at a dose of 300 µL/mouse. The mice were challenged with a dose of 1000 TCID50 per mouse on day 0. After the challenge, the medicament was administered by atomization at 6 h, day 2, day 4, and day 6. The doses of each administration were 0.2, 1, and 5 µg/g. The mice were weighed for 14 consecutive days to observe the survival and survival status of the mice. Survival rate analysis (**FIG. 4B** and **FIG. 4C**) showed that PR8-infected mice began to die on the 8th day after infection, and all died on the 13th day. The TFF2-IFNα2-Fc fusion protein was able to protect about 40% of mice from influenza death. As shown in **FIG. 4B** and **FIG. 4C****,** the weight of PR8-infected mice continued to decrease, and most mice lost more than 20-30% of their weight on the 10th day. The fusion protein group began to gradually increase its weight on the 11th day, while the IFNα2-TFF2-Fc 0.5 µg/g group kept decreasing. Compared with TFF2-IFNα2-Fc, the overall protective effect of IFNα2-TFF2-Fc was not good, which may be due to its induction of a higher inflammatory response, which reduced the protective effect.

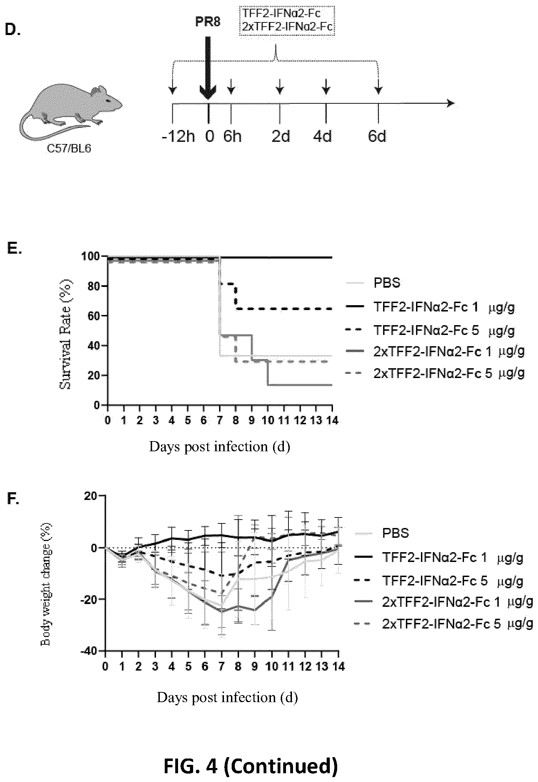

. In the prevention experiment of animals with fusion protein, influenza virus H1N1 strain PR8 (in the P2 laboratory) was used to infect C57 mice via intranasal administration. The fusion protein was administered 12 h prior to infection (FIG. 4D). Before infection, 0.5 g of Tribromoethanol + 1 mL of 2-methyl-2-butanol + 39 mL of water was used to prepare a 40 mL solution to anesthetize mice at a dose of 300 µL/mouse. The mice were challenged with a dose of 500 TCID50/mouse on day 0. After the challenge, the medicament was administered by atomization at 6 h, day 2, day 4, and day 6. The doses of each administration were 1 µg/g and 5 µg/g. The mice were weighed for 14 consecutive days to observe the survival and survival status (weight changes) of the mice. Survival rate analysis **(****FIG.** 4E) showed that PR8-infected mice began to die on the 7th day after infection, and the survival rate of mice in the PBS group was 30%. The TFF2-IFNα2-Fc fusion protein protected all mice from influenza death at a dose of 1 µg/g. As shown in **FIG.** 4F, the weight of PR8-infected mice continued to decrease, and on Day 7, most mice lost more than 10-30% of their weight. The fusion protein TFF2-IFNα2-Fc had a maximum weight loss of about 10% at a dose of 5 µg/g, and basically no weight loss at a dose of 1 µg/g. The 2xTFF2-IFNα2-Fc had no protective effect at doses of 1 µg/g and 5 µg/g, which indicates that the 2xTFF2 structure in 2xTFF2-IFNα2-Fc would affect the function of IFNα2 and thus affect the protective effect.

**.** The above results prove that the fusion protein TFF2-IFNα2-Fc fusion protein can produce significantly better effects than TFF2 alone, IFNα2 alone, and other forms of fusion proteins in preventing and protecting mice from weight loss and death caused by influenza virus.

. All references cited herein are incorporated by reference in their entirety as though each reference was individually incorporated by reference. In addition, it should be understood that after reading the contents of the present application, those skilled in the art may make various changes or modifications to the present application, and these equivalent forms also fall within the scope defined by the appended claims of the present application.

### Appendix: Sequence information

| SEQ ID NO: | Information | SEQ ID NO: | Information |
|---|---|---|---|
| 1 | TFF2-G4S-IFNα2-Fc mRNA coding sequence | 7 | Human TFF2 nucleotide sequence |
| 2 | TFF2-G4S-IFNα2-Fc amino acid sequence | 8 | Human TFF2 amino acid sequence |
| 3 | 2xTFF2-G4S-IFNα2-Fc mRNA coding sequence | 9 | Human IFNα2 nucleotide sequence |
| 4 | 2xTFF2-G4S-IFNα2-Fc amino acid sequence | 10 | Human IFNα2 amino acid sequence |
| 5 | IFNα2-G4S-TFF2-Fc mRNA coding sequence | 11 | Mouse TFF2 nucleotide sequence |
| 6 | IFNα2-G4S-TFF2-Fc amino acid sequence | 12 | Mouse TFF2 amino acid sequence |
| | | 13 | Mouse IFNα2 nucleotide sequence |
| | | 14 | Mouse IFNα2 amino acid sequence |

## Claims

1. A fusion protein comprising one or more fusion units, each fusion unit comprising:
(a) a trefoil factor 2 (TFF2) element, wherein the TFF2 element comprises a TFF2 peptide or an active fragment thereof;
(b) an interferon α2 (IFNα2) element, wherein the IFNα2 element comprises an IFNα2 peptide or an active fragment thereof,
wherein the TFF2 element and the IFNα2 element are fused at a molecular ratio of 1:1, and in each fusion unit the TFF2 element is located at the N-terminus of the IFNα2 element.

2. The fusion protein according to claim 1, wherein the TFF2 peptide or an active fragment thereof is derived from humans, primates, rodents (e.g., mice, rats, guinea pigs, hamsters), dogs, cats; and/or
the TFF2 peptide or an active fragment thereof comprises an amino acid sequence selected from the group consisting of SEQ ID NO: 8 and SEQ ID NO: 12, or an active fragment thereof (e.g., an amino acid sequence that has at least 80% sequence identity with SEQ ID NO: 8 and SEQ ID NO: 12 and has TFF2 activity); and/or
the TFF2 peptide or an active fragment thereof is encoded by a nucleic acid molecule comprising a nucleotide sequence selected from the group consisting of SEQ ID NO: 7 and SEQ ID NO: 11, or an active fragment thereof (e.g., a nucleic acid molecule having at least 80% sequence identity with SEQ ID NO: 7 or SEQ ID NO: 11 and capable of encoding an active TFF2 peptide).

3. The fusion protein according to claim 1, wherein the IFNα2 peptide or an active fragment thereof is derived from humans, primates, rodents (e.g., mice, rats, guinea pigs, hamsters), dogs, cats; and/or
the IFNα2 peptide or an active fragment thereof comprises an amino acid sequence selected from the group consisting of SEQ ID NO: 10 and SEQ ID NO: 14, or an active fragment thereof (e.g., an amino acid sequence that has at least 80% sequence identity with SEQ ID NO: 10 and SEQ ID NO: 14 and has IFNα2 activity); and/or
the IFNα2 peptide or an active fragment thereof is encoded by a nucleic acid molecule comprising a nucleotide sequence selected from the group consisting of SEQ ID NO: 9 and SEQ ID NO: 13, or an active fragment thereof (e.g., a nucleic acid molecule having at least 80% sequence identity with SEQ ID NO: 9 or SEQ ID NO: 13 and capable of encoding an active IFNα2 peptide).

4. The fusion protein according to claim 1, wherein the fusion protein further comprises a linker that connects the TFF2 element and the IFNα2 element and/or the constituent peptide segments in the element,
for example, the linker is a flexible linker comprising n amino acid residues, where n is an integer of 2-300;
for example, the linker is a glycine or glycine/serine linker, such as Gₙ, (GS)ₙ, (GGS)ₙ, (GGGS)ₙ, (GGGGS)ₙ or (GGGGGS)ₙ, wherein n is an integer of 1, 2, 3, 4, 5, 6, 7, 8, 9 or 10.

5. The fusion protein according to claim 1, wherein the fusion protein comprises one or more TFF2 peptides arranged continuously or intermittently and/or one or more IFNα2 peptides arranged continuously or intermittently.

6. The fusion protein according to claim 1, wherein the fusion protein further comprises an Fc region, wherein the Fc region does not contain a mutation, or comprises one or more mutations that reduce antibody-mediated ADCC and CDC activity, such as amino acid mutations D265A and N297G according to EU numbering; and/or
the fusion protein further comprises a signal peptide, for example, selected from tPA2 signal peptide, TFF2 signal peptide, IL-2 signal peptide, bPRL signal peptide, CD33 signal peptide; and/or
the fusion protein further comprises a label, for example, a label for purification, detection, and localization, such as selected from fluorescent labels, non-radioactive nuclide labels, biotin labels, phosphorylation modification labels, and peptide tags.

7. The fusion protein according to claim 1, wherein the fusion protein has an amino acid sequence of SEQ ID NO: 2 or has at least 80% sequence identity thereto; and/or
the fusion protein is encoded by a nucleic acid molecule having a nucleotide sequence of SEQ ID NO: 1 or a nucleic acid molecule having at least 80% sequence identity thereto.

8. An isolated nucleic acid molecule or a construct or vector comprising the nucleic acid molecule, wherein the nucleic acid molecule encodes the fusion protein according to any one of claims 1 to 7.

9. The nucleic acid molecule, construct or vector of claim 8, wherein the nucleic acid molecule has a nucleotide sequence of SEQ ID NO: 1 or has at least 80% sequence identity thereto; and/ or
the nucleic acid molecule encodes an amino acid sequence of SEQ ID NO: 2 or a polypeptide having at least 80% sequence identity thereto; and/or
the vector is selected from a viral vector, an mRNA vector, and a DNA vector.

10. A cell comprising the fusion protein of any one of claims 1 to 7, or the nucleic acid molecule, construct, or vector of claim 8 or 9.

11. A composition comprising the fusion protein of any one of claims 1 to 7, the nucleic acid molecule, construct, or vector of claim 8 or 9, or the cell of claim 10; and a carrier.

12. Use of the fusion protein of any one of claims 1-7, the nucleic acid molecule, construct, or vector of claim 8 or 9, the cell of claim 10, or the composition of claim 11 in the preparation of a medicament for preventing and/or treating a viral infectious disease.

13. The use according to claim 12, wherein the viral infectious disease is selected from acute viral infection, such as respiratory viral infection and enteroviral infection; and/or
the viral infectious disease is caused by one or more viruses selected from the group consisting of coronavirus, influenza virus, rhinovirus, adenovirus, parainfluenza virus, respiratory syncytial virus, coxsackievirus , echovirus, and novel enterovirus.

14. The use according to claim 12, wherein the medicament is administered as a prophylactic drug before a viral infection occurs to prevent the occurrence of a viral infection or reduce the severity of a subsequent viral infection;
the medicament is administered as a therapeutic drug after a viral infection occurs to reduce the severity of the viral infection and disease; and/or
the medicament is used as both a preventive drug and a therapeutic drug, and is administered continuously or intermittently before and after the occurrence of a viral infection.

15. The use according to claim 12, wherein the dosage form of the medicament is suitable for an administration method selected from the group consisting of respiratory tract atomization inhalation, nasal drops, spray, oral administration, intramuscular injection and/or intravenous administration; and/or
the medicament is suitable for use alone or in combination with other antiviral drugs, immune drugs or viral therapies.
